# EUROPEAN PATENT APPLICATION

(11) **EP 1 586 336 A1**
(43) Date of publication of application: **19.10.2005**
(21) Application number: 04076073.8
(22) Date of filing: 06.04.2004
(51) Int. Cl.: A61L 15/18, A61L 15/32

(54) **Compositions containing copper salts and soy products**

(71) Applicant: JOHNSON & JOHNSON GmbH, 40474 Düsseldorf (DE)
(72) Inventor: Oddos, Thierry, 92190 Meudon (FR); Von Stetten, Otto, 52072 Aachen (DE)
(74) Representative: Weber-Bruls, Dorothée

(57) **Abstract**

The invention relates to compositions containing copper salts and soy products that can be used to prevent or treat conditions that are caused by the decomposition products of lipases and proteases, in particular to prevent or treat diaper rash.

## Description

### Field of the Invention

The invention relates to compositions containing copper salts and soy products that can be used to prevent or treat conditions that are caused by the decomposition products of substrates by intermediation of lipases and proteases, in particular to prevent or treat diaper rash.

### Background of the Invention.

The products resulting from decomposition of substrates through the action of lipase and protease enzymes are causative to a number of dermatological conditions such as redness, irritation and may even result in inflammatory conditions. Often lipases and proteases are both present whereby these conditions are aggravated. Situations where both enzymes are active together are, for example, the decomposition of perspiration products and in diaper rash. Especially the latter is a condition where both enzymes play a key role.

Diaper rash, also referred to as diaper dermatitis, is a frequently occurring condition in babies and infants resulting in irritation and inflammation and causing discomfort. It also occurs in incontinent adults.

Diaper rash in general is defined as a kind of inflammatory skin condition in the diaper area of an individual caused by factors such as moisture, occlusion and chafing in combination with prolonged contact with urine, faeces or both. In its initial stages, diaper rash is a kind of contact dermatitis which can evolve to more serious conditions. The combined activity of enzymes, especially lipases and proteases is believed to play a causative role in the effects associated with diaper rash. Moreover the lipases and protease enzymes from faeces are activated by maceration with urine. The urea present in urine is broken down to ammonia which increases the pH This in turn activates certain digestive enzymes, in particular the lipases and proteases. The former decompose the triglycerides in the sebum into fatty acids which are believed to play an active role in the diaper rash condition. This is aggravated by microbiological contamination both of bacterial and fungal nature, e.g. Candida albicans.

Usually prevention of diaper rash is achieved by isolation of the skin from the enzymes by application of a barrier material that isolates the skin from urine and faeces, in particular by using suitable ointments which create a kind of protective lipophilic film. A traditional barrier material is composed of petrolatum (petroleum jelly) usually in combination with zinc oxide. The latter is a mild astringent and in itself contributes to the barrier function. Zinc moreover is known to inhibit the lipase enzymes.

However, the protective film may be penetrated by the enzymes thus leading to the adverse effects associated with diaper rash. With zinc salts lipases may be inhibited, but the activity of proteases is not affected.

Furthermore, diaper rash has been treated by promoting dryness e.g. by the use of baby powder. Also certain active agents have been used such as anti-bacterials, anti-fungals and anti-inflammatory agents, usually incorporated in ointments salves or creams.

Diaper rash may also be treated by using certain clays that may absorb the lipase and protease enzymes but the adsorption of such special clays is non-specific and reversible e.g. by substances with higher affinity to clay or by change in the chemical environment such as pH.

There is a general need to provide means to better deal with the conditions related to the presence of decomposition products of lipases and proteases, in particular to deal with conditions in relation to the decomposition of the components present in perspiration. Additionally there is a more specific need for finding new and better means to deal with the effects of diaper rash. The compositions of the invention containing both copper salts and soy products are aimed at meeting these needs.

Soy products as well as their use have been described in the prior art. WO-01/34099 describes the use of non-denatured soy product containing compositions for depigmentation, evening out skin tone and skin texture, skin firming and care of the skin. US-6,555,143 discloses compositions and methods that relate to legume products and in particular to soy products for regulating firmness of the skin, hair or nails; cleansing the skin, hair or nails; reducing and/or delaying hair or nail growth; and a number of other useful applications. EP-A-1236465 describes legume products having trypsin inhibitory activity, in particular soy products, having reduced microbial content and the use thereof in compositions for application on the skin, nails and hair.

### Summary of the Invention

The present invention relates to compositions containing one or more copper salts and one or more soy products. The compositions of the invention in particular are for topical use.

In a particular aspect there is provided a composition, in particular a topical composition, containing an amount of one or more copper salts and an amount of one or more soy products, said amounts being effective to block the activity of lipases and proteases.

In a further aspect there is provided a composition containing
(a) from 0.001% to 5% of one or more copper salts; and
(b) from of 0.01% to 5% of one or more soy products.

Preferably the invention concerns compositions as specified herein wherein the soy product is non-denatured soy product.

In another aspect, the invention features the use, in particular the topical use, of a composition as specified herein for preventing and/or treating the adverse effects caused by the decomposition products from lipases and proteases, more specifically for preventing and/or treating the effects associated with diaper rash, or further specifically for preventing and/or treating the adverse effects associated with the decomposition products of perspiration.

The invention further provides a topical formulation for treating and/or preventing diaper rash, said formulation comprising a composition as specified herein.

The invention also provides an anti-perspirant formulation comprising an anti-perspirant active ingredient and a composition as specified herein.

In still another aspect there is provided the use, in particular the topical use, of a composition as specified herein as a combined blocker of the activity of lipases and proteases. In still another aspect the invention relates to the use, in particular the topical use of a composition as specified herein to prevent or treat inflammation.

In a further aspect the present invention relates to a method of treating and/or preventing diaper rash and diaper dermatitis caused by the prolonged contact of human skin with body waste. The method of the present invention requires the topical application of a composition suited for topical application, comprising a combination of one or more copper salts and one or more soy products.

### Detailed Description of the Invention

The copper salts used in the compositions in accordance with the present invention are any skin-compatible copper salts such as, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, *p*-aminosalicylic, and the like acids. The term "addition salt" as used herein also comprises the solvates which the copper salts are able to form. Such solvates are for example hydrates, alcoholates and the like. Of particular interest are copper salts that generate free copper ions, i.e. copper salts that readily split off copper ions.

Preferred are copper acetate, the copper halogenides, in particular copper chloride and bromide, copper sulfate and copper nitrate. It has been found that such type of copper salts possess particularly effective lipase inhibitory properties.

The compositions of the present invention contain soy products that may be in the form of a fluid (e.g. soymilk) or a solid (e.g. a soybean powder or soymilk powder). What is meant by "soy product" is a substance derived from the soybean, containing the ingredients naturally found in soybeans, at the relative concentrations as found in the beans. In preferred embodiments, the soy product is a non-denatured soy product. The latter is a soy product which has been obtained by processes that leave the active proteins intact by carefully controlling the process parameters such as the temperature, the extraction media. This can be measured, for example, by the presence of intact soybean trypsin inhibitor (STI) protein.

In another embodiment, the soy product is soymilk. One way to make soymilk is to soak the soybeans in deionized or purified water for several hours, and grind them after they were fully hydrated, with the addition of small quantities of water. (The grinding process allows the soybean milk to be extracted). After collection, the soybean milk may be filtered to remove any residual parts of the bean husk. The soymilk used in this invention can be fresh soymilk as described above, or may be made from soybean powder and water. The soybean powder is milled from soybeans and may also be lyophilized, spray dried, or freeze-dried and the resulting soymilk may or may not be filtered. Such prepared soymilk may have from about 1 to about 90% by weight dry soybean powder. Another example is the use of soymilk powder, made from lyophilized, spray dried or freeze-dried soymilk, with the addition of water and finished with or without filtration or homogenization.

Other methods of soybean extraction could also be used to create the active ingredients used in this invention. For example, but not limited to, the active ingredients could be extracted from ground soybeans using ethanol/water mixtures, followed by the removal of the ethanol from the extract, in such ways that the protease inhibitory activity of the soybean will be retained.

The compositions of the present invention may contain from about 1% to about 99%, by weight, of the soy product. For example, when a liquid soy product (e.g., soymilk) is used, the composition may contain from about 50% to about 99%, by weight, (e.g., from about 70% to about 99%) of the liquid soy product. For example, when a solid soy product (e.g., soybean powder or soymilk powder) is used, the composition may contain from about 1% to about 50%, by weight (e.g., from about 2% to about 30%, by weight) of the solid soy product. Compositions that comprise solid soy products may also comprise water (e.g., distilled water or water contained within soymilk) to form a liquid base to the composition (e.g., to form a cream, lotion or gel). Such compositions may comprise from about 50% to about 98% by weight (e.g., from about 70% to about 98%, by weight) of water.

The soy products useful in this invention may be produced from all soybean species, regardless of their geographic origin, sun exposure, harvest time and the like. However, specific strains, geographic origins or growth conditions might be preferred.

For example, but not limited to, soybean strains particularly rich in their trypsin inhibitor (e.g. STI, LTI, BBI) content or growth conditions that result in trypsin inhibitor enrichment in the bean, ought be preferred. It should be noted that the soy products useful in the compositions of this invention have a distinctive odor, which may be tolerable in some cultures, but is undesired in others. If necessary, the odor of the compositions of this invention may be reduced by using soybean products derived from specific strains of soybeans known to produce reduced odor, including, but not limited to, lipoxygenase-2-deficient beans and those having modified sugar profile, and the like. A process to reduce oxygen levels in the formulation may also reduce the odor. Various masking agents or fragrances may also be used to mask the odor.

Preferred for use in the compositions of the present invention are non-denatured soy products. These are preferably decontaminated as described in EP-1236465, for example by gamma irradiation of non-denatured soymilk powder, preferably at a dose of about 10 kGy.

The compositions of this invention may contain further stabilizing components. The latter may for example comprise one or more components selected from the group consisting of one or more antioxidants and preservatives.

The compositions of this invention may contain one or more preservatives. Preservatives are useful for substantially preventing microbial decomposition. Examples of preservatives include phenoxyethanol and parabens such as methylparaben, ethylparaben, and propylparaben. Other examples of preservatives are listed on pages 1654- 55 of the International Cosmetic Ingredient Dictionary and Handbook, eds. Wenninger and McEwen (CTFA, 7th ed., 1997), hereinafter referred to as the "Cosmetic Handbook." The compositions may comprise from about 0.01% to about 20%, by weight (more preferably, from about 0.5% to about 5%, by weight) of preservative. Microbial contamination can also be eliminated by gamma irradiation or microfiltration or by brief heat treatments that do not result in the elimination of protease inhibitory activity.

Antioxidants and/or chelating agents may also be used to increase shelf life and stability of the compositions. Antioxidants may be added both for formulation stabilization and for biological efficacy. Antioxidant compounds and their derivatives include, but are not limited to, water-soluble antioxidants such as sulfhydryl compounds and their derivatives (e.g., sodium metabisulfite and N-acetyl-cystein), lipoic acid and dihydrolipoic acid, resveratrol, acetyl-cysteine (Ineferine™) or lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g. retinol and retinyl palmitate), tocopherols (e.g., tocopherol acetate), tocotrienols, and ubiquinone. Natural extracts containing antioxidants suitable for use in the compositions of this invention, include, but are not limited to, extracts containing flavonoids and isoflavonoids and their derivatives (e.g., genistein and diadzein), extracts containing resveratrol and the like. Examples of such natural extracts include grape seed, green tea, pine bark, propolis, and legume extracts. Other examples of antioxidants may be found on pages 1612-13 of the Cosmetic Handbook. The compositions of the present invention may comprise the antioxidant in an amount of from about 0.001% to about 20%, by weight (e.g., from about 0.01% to about 10% by weight) of the composition.

Thickening agents (e.g., thickeners or viscosity enhancing agents) may be utilized in the compositions of this invention to alter their viscosity. The desired viscosity of the composition will depend upon the intended use (e.g., as a bath product, cream, lotion, or gel). For example, in applications such as bath or wash products, the viscosity of the composition should be relatively low, similar to an aqueous solution. Application as a cream, lotion, or gel will have slightly higher viscosity (e.g., between about 100 cPs and 100,000 cPs).

Thickening agents that can be added to the compositions of this invention to alter viscosity include polymers such as polyacrylates (e.g., polyacrylamide). Other examples of viscosity modifying agents are listed on pages 1692-97 of the Cosmetic Handbook. To achieve the appropriate viscosity, compositions of the present invention may comprise from about 0.01% to about 20%, by weight (e.g., from about 0.1% to about 5%, by weight) of a thickening agent.

The compositions of the invention can be prepared by adding the aqueous formulations of the salts to the soy component or vice versa. The compositions of the present invention therefore are mostly of aqueous nature.

In the compositions of the present invention the w/w ratio of the total amount of soy to the total amount of copper salts products may vary, but in particular is in the range of about 50 : 1 to 1 : 1, further in particular from 30 : 1 to 2 : 1, still further in particular from 20 : 1 to 5 : 1, These w/w ratios relate to the total amount of dry soy product and dry salts.

The compositions may contain further ingredients or additives such as active ingredients, surfactants, emulsifiers, consistency factors, conditioners, emollients, skin caring ingredients, moisturizers, lubricants, fillers, binding agents, anti-oxidants, preservatives, fragrances and the like.

The soy product preferably is used in the compositions of the invention at concentrations from 0.001% to 10% and preferably from 0.1% to 5%, more preferably from 0.5 % to 5 % further preferably from 1% to 3%. The total weight-% in the compositions of all copper salts taken together may be between about 0.001% to 5%, in particular in the range of from 0.001 to 3%, more in particular in the range of from 0.005 to 2%, preferably from about 0.01% to 1%, more preferably from 0.1 % to 1%, or further preferably from 0.2% to 0.5%.

Unless indicated otherwise, all percentages in the preceding and following paragraphs are w/w percentages.

The formulations according to the present invention can be prepared by mixing the appropriate ingredients. It is also possible to make premixes and to add ingredients or other premixes. In a preferred method of preparation, the compositions of this invention are emulsion-based, in particular oil-in-water emulsions and are made by preparing an aqueous phase containing all hydrophilic components, an oil phase containing all lipophilic components, and subsequently adding the oil phase to the aqueous phase as to prepare an emulsion. Preferably, a premix of the soy product in some water is added after the formation of the emulsion.

The compositions according to the invention may be used in topical formulations that may take a variety of forms, which will depend upon the nature of the end product used. The compositions may be in the form of a solution, a hydrophilic lotion, an ointment, a cream or a gel. The formulations may also be, for example, in the form of oil-in-water, water-in-oil or multiple emulsions or foaming products.

The compositions of the invention may also be formulated into formulations for anti-perspirant use and more in particular they may find use in deodorant formulations. These formulations may take any of the forms used in these type of applications such as sprays, sticks, roll-ons, etc.

The compositions of this invention comprise ingredients that inhibit both the lipase and protease enzymes, i.e. copper salts to inhibit lipases, soy products inhibit proteases.

Moreover the copper salts act additionally as anti-microbial agents. Both ingredients moreover act in a synergistical manner.

Therefore the compositions of the invention are particularly useful in the prevention and treatment of the effects or the prevention and treatment of the conditions associated with the presence of the decomposition products of substrates decomposed by lipase and protease enzymes. The compositions in particular are useful to prevent or treat conditions such as diaper rash, more specifically to prevent or treat the effects associated with diaper rash. Additionally the compositions of the invention may find use in the prevention and treatment of inflammation caused by products that result from the decomposition of substrates by the intermediation of lipase and protease enzymes.

Preferred are compositions in accordance with the present invention wherein the soy products are non-denatured soy products. Such compositions are particularly effective in the uses mentioned herein above, and in particular in the use to prevent or treat the effects associated with diaper rash.

The following example is meant to illustrate the invention and not to limit it thereto.

### Examples

### Example 1

| **Component INCI Name** | **%** |
|---|---|
| Aqua | 51.836 |
| Carbomer | 0.100 |
| Acrylates / C 10-30 alkyl acrylate crosspolymer | 0.060 |
| Sodium hydroxide | 0.050 |
| Tromethamine | |
| Glycerin | 18.000 |
| Disodium EDTA | 0.100 |
| Methyl paraben | 0.350 |
| Isopropyl Myristate 10% / Cetearyl Ethylhexanoate 90% | 8.000 |
| Butyrospermum parkii | 1.500 |
| Cetearyl alcohol | 4.000 |
| Glyceryl Stearate SE | 4.500 |
| Potassium Cetyl phosphate | 0.500 |
| Beta sitosterol | 0.500 |
| Vitis vinifera | 2.000 |
| Ascorbyl palmitate 25% / Lecithin 70%/ tocopherol 5 % | 0.004 |
| Dimethicone | 1.000 |
| Propyl Paraben | 0.200 |
| Acrylates copolymer 50%/ Glycerin 50% | 1.000 |
| Glycine Soya | 2.000 |
| Sodium Benzoate | 0.200 |
| Phenoxyethanol | 0.800 |
| Chlorhexidine digluconate 20%/Aqua 80% | 0.250 |
| Copper acetate | 0.300 |
| Panthenol 75% / aqua 25% | 2.750 |
| Total | 100.000 |

### Example 2

### Chymotrypsin inhibition

Chymotrypsin inhbition was assessed in a specific in vitro assay based on the hydrolysis of N-Benzoyl-L-Tyrosine Ethyl Ester (BTEE), a specific chromogenic substrate as described below :

BTEE + H2O _{Chymotrypsin}> N-Benzoyl-L-Tyrosine + Ethanol

The reaction is performed in Tris HCl buffer pH8.0 in the presence of calcium Chloride. The hydrolysis of BTEE is followed by an increase in absorbance at 246 nm. Addition of increasing concentration of soy extract decreased the activity of the protease in a dose dependent manner as shown in figure 1 :

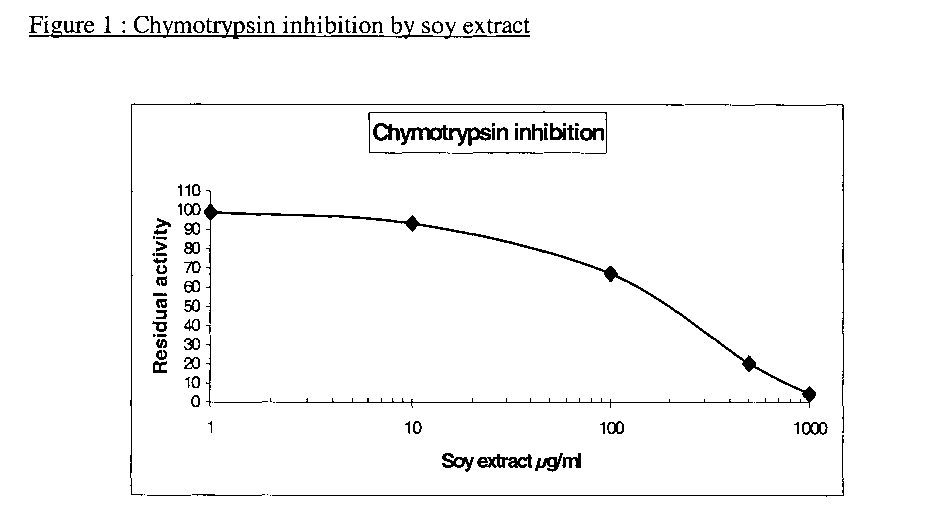

### Lipase inhibition

Evaluation of the ability of compounds to inhibit lipase activity was performed in vitro with a titrimetric method. Lipase is incubated with a refined olive oil emulsion with a TrisHCl buffer at pH 7.7. The activity of lipase on triglyceride leads to the production of free fatty acid that are then titrated with sodium hydroxyde 1N.

Triglyceride + H₂O Lipase > Diglyceride + Fatty Acid

For each copper salt the concentration giving 50% of enzyme inhibition was calculated and summarized in the table below.

**Table :**

| Lipase inhibition by copper salts | |
|---|---|
| Inhibition of lipase activity | |
| **Compound** | **IC50 µg/ml** |
| Cu Acetate | 364 |
| Cu Chloride | 531 |
| Cu Gluconate | 3182 |
| Copper peptide | No activity |

### Anti-inflammatory properties of copper chloride and soy extract

Soy and copper chloride efficacy on diaper rash inflammation was evaluated on an in-vitro diaper rash model where reconstituted epidermis were treated with chymotrypsin and lipase to induce an inflammatory response which was assessed by the release in the medium of interleukin 8, an inflammatory mediator. In that model, lipase and chymotrypsin treatment of the epidermis increased the production of IL8. This pro-inflammatory response was markedly inhibited by the co-treatment of the epidermis with soy and copper chloride.

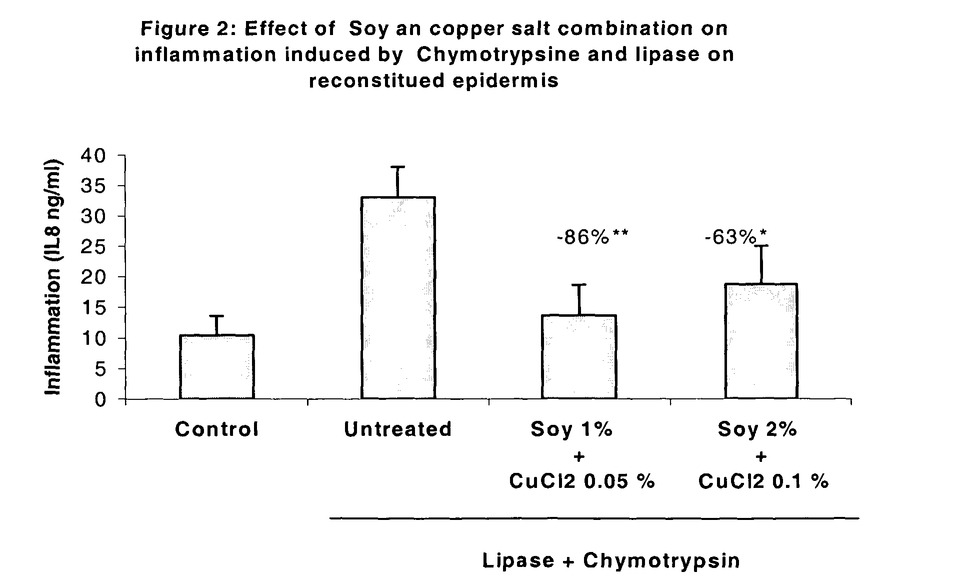

## Claims

1. A composition containing one or more copper salts and one or more soy products.

2. The composition of claim 1, in particular a topical composition, containing an amount of one or more copper salts and an amount of one or more soy products, said amounts being effective to block the activity of lipases and proteases.

3. The composition of claim 1, said composition containing
(a) from 0.001% to 5% of one or more copper salts; and
(b) from of 0.01% to 5% of one or more soy products.

4. The composition according to any of claims 1 - 3 wherein the soy product is non-denatured soy product.

5. The use, in particular the topical use, of the composition as claimed in claims 1 - 4 for preventing and/or treating the adverse effects caused by the decomposition products from lipases and proteases, more specifically for preventing and/or treating the effects associated with diaper rash, or further specifically for preventing and/or treating the adverse effects associated with the decomposition products of perspiration.

6. A topical formulation for treating and/or preventing diaper rash, said composition comprising a composition as claimed in claims 1 - 4.

7. An anti-perspirant formulation comprising an anti-perspirant active ingredient and a composition as claimed in claims 1 - 4.

8. In still another aspect there is provided the use , in particular the topical use, of a composition as claimed in claims 1 - 4 as a combined blocker of the activity of lipases and proteases.
